(19) Europäisches Patentamt
European Patent Office
Office européen des brevets

(11) Veröffentlichungsnummer: **0 234 262 B1**

# (12) EUROPÄISCHE PATENTSCHRIFT

(45) Veröffentlichungstag der Patentschrift: **08.08.90**

(51) Int. Cl.⁵: **A61K 31/52** // (A61K31/52, 31:44)

(21) Anmeldenummer: **87100747.2**

(22) Anmeldetag: **20.01.87**

(54) **Arzneimittelkombination.**

(30) Priorität: **10.02.86 DE 3604149**

(43) Veröffentlichungstag der Anmeldung: **02.09.87 Patentblatt 87/36**

(45) Bekanntmachung des Hinweises auf die Patenterteilung: **08.08.90 Patentblatt 90/32**

(84) Benannte Vertragsstaaten: **AT BE CH DE FR GB IT LI LU NL SE**

(56) Entgegenhaltungen:
**CHEMICAL ABSTRACTS, Band 99, Nr. 1, 4. Juli 1983, Seite 31, Zusammenfassung Nr. 284q, Columbus, Ohio, US; K. KATSUMOTO et al.: "Experimental comparison of the efficacy of various agents on the enhancement of myocardial protection", & JPN. CIR. J. 1983, 47(3), 356-62anamides and N,N-di-n-hexylocatanamides", & COM- NAZ. ENERG. NUCL., [RAPP. TEC.] CN 000**
**CHEMICAL ABSTRACTS, Band 97, Nr. 25, 20. Dezember 1982, Seiten 7-8, Zusammenfassung Nr. 207583r, Columbus, Ohio, US; N. TSUSHIMA et al.: "Pharmacologial effects on the microcirculation of the human conjunctiva", & INT. CONGR. SER.-EXCERPTA MED. 1982, 578(Basic Aspects Microcirc.) 85-94n-hexylocatanamides", & COM- NAZ. ENERG. NUCL., [RAPP. TEC.] CN 000**

(73) Patentinhaber: **Dr. Rentschler Arzneimittel GmbH & Co., Postfach 320 Mittelstrasse 18, D-7958 Laupheim(DE)**

(72) Erfinder: **Nandi, Kumaresh, Dr. Dipl.-Chem., Theodor-Heuss-Str. 1, D-7958 Laupheim(DE)**
Erfinder: **Fischer, Helga, Bachstr. 17, D-7938 Oberdischingen(DE)**
Erfinder: **Herrmann, Wilfried, Dr., Kurt-Schumacher-Str. 2, D-7958 Laupheim(DE)**
Erfinder: **Köhne, Hans, Dr. Dipl.-Chem., Rotbachweg 7, D-7959 Obersulmetingen(DE)**
Erfinder: **Lahr, Wolfgang, Silcherweg 29, D-7958 Laupheim(DE)**
Erfinder: **Schmersahl, Hein Uwe, Dr., Friedhofweg 6, D-7930 Ehingen 17(DE)**
Erfinder: **Walch, Hatto, Dr., Weissdornweg 10, D-7958 Baustetten(DE)**

(74) Vertreter: **Dipl.-Ing. Schwabe, Dr. Dr. Sandmair, Dr. Marx, Stuntzstrasse 16 Postfach 86 02 45, D-8000 München 86(DE)**

EP 0 234 262 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die Erfindung betrifft pharmazeutische Kombinationspräparate zur Behandlung von Durchblutungsstörungen im weitesten Sinne, die ursächlich oder symptomatisch verschiedene Krankheitsbilder bestimmen, die bislang mit jeweils wirkungsspezifischen Arzneimitteln, behandelt wurden.
Dihydropyridine der allgemeinen Formel

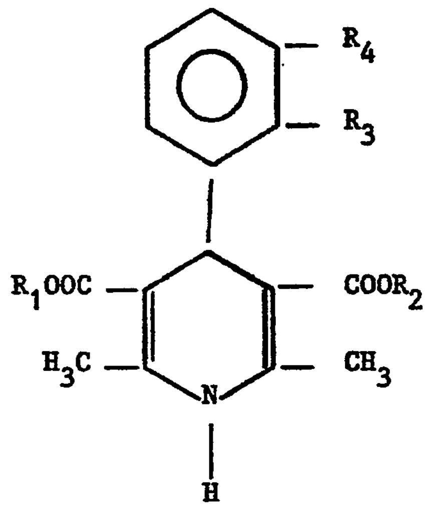

in welcher für
$R_1$ eine $CH_3$-O-$CH_2$-$CH_2$- oder $CH_3$-Gruppe, für $R_2$ eine

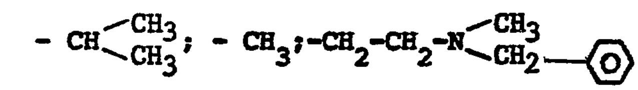

oder $CH_3$-$CH_2$-Gruppe; für $R_3$ H, $NO_2$ oder Cl und für
$R_4$ $NO_2$, H oder Cl
stehen kann, mit calciumantagonistischer Wirkung, insbesondere Nifedipin, Nimodipin, Nitrendipin, Nicardipin und Felodipin, sind bekannt und werden bei der Therapie der koronaren Herzkrankheit (KHK) und/oder des Bluthochdrucks (Hypertonie) verwendet. Diese ausgewählten Dihydropyridine allein hemmen selektiv den Calciumeinstrom durch die Zellmembran an der glatten Muskulatur der Gefäße, einschließlich der Herzkranzgefäße, ohne daß die Calciumionenkonzentration im Blutserum beeinflußt wird. Daraus resultiert die Wirksamkeit speziell bei der KHK, der Hypertonie und bei spastischen Zuständen der glatten Muskulatur, beispielsweise bei Asthma, Uterusspasmen u.s.w. (siehe A. Fleckenstein, H. Roskamm, "Calcium-Antagonismus", Springer Verlag, 1980).

Pentoxifyllin ist gleichfalls seit geraumer Zeit bekannt. Seine Herstellung geht insbesondere aus der DE-PS Nr. 12 35 320 hervor. Pentoxifyllin wird bei peripheren- und Gehirndurchblutungsstörungen angewendet. Es besitzt kein ausgeprägtes hämodynamisches (die Blutströmung betreffend) und spasmolytisches Wirkprofil, wie etwa die vorgenannten Dihydropyridine. Es verbessert primär die Erythrozytenflexibilität, vermindert die Erythrozytenaggregationsneigung durch Erhöhung des cAMP (cyclisches Adenosinmonophosphat) durch Hemmung der Phosphodiesterase und bewirkt dadurch eine Verminderung der Blutviskosität. Zusätzlich wird die Thrombozytenaggregation vermindert, das Fibrinogen und die Plasmaviskosität gesenkt. [R. Müller, Therapiewoche 30, 2440 - 2451 (1980), Aviado, Porter, Pharmacotherapy Vol.4, Nr. 6 (1984), Dettelbach, Aviado, Journ. of Clin. Pharm. 1985, S. 8 - 26].

Die erfindungsgemäß verwendeten Dihydropyridine zeigen zwar gleichfalls einen durchblutungsfördernden Effekt, der auf einer Gefäßerweiterung beruht. Für die Gewebsversorgung ist jedoch eine optimale Mikrozirkulation entscheidend, die allein durch Gefäßerweiterung nicht erzielt werden kann. Insofern erzielt die alleinige Anwendung von Dihydropyridinen keinen ausreichenden therapeutischen Nutzen.

Darüber hinaus ist dieser Effekt wegen der erforderlichen hohen Dosen und den damit verbundenen Nebenwirkungen nicht nutzbar.

Zusätzlich kann als Umkehreffekt das sogenannte steal-Phänomen auftreten, das heißt, eine zusätzliche Mangelversorgung bereits unterversorgter Gewebsareale mit Blut. Darüber hinaus verursachen Dihydropyridine schon bei üblichen therapeutischen Dosen erhebliche Nebenwirkungen, wie z. B. Kopfschmerz, Beinoedeme, Flush, Reflextachykardie. Bei höheren Dosen ist zusätzlich mit einem stärkeren

Blutdruckabfall unter die gewünschte Norm zu rechnen, der die Mangelversorgung infolge des verminderten Perfusionsdrucks, insbesondere im cerebralen Bereich, verstärkt.

Pentoxifyllin besitzt keine ausgeprägte hämodynamische Wirkung, so daß diese Substanz allein bei der Behandlung der koronaren Herzkrankheit und Hypertonie keine Anwendung findet. Pentoxifyllin wird in Tagesdosen bis zu ca. 1.600 mg peroral verabreicht, in angezeigten Fällen sogar weit darüber hinaus (z. B. bei gleichzeitiger peroraler und parenteraler Therapie), bzw. in Einzeldosen bis zu 600 mg. Diese therapeutisch notwendigen Hochdosen führen bekanntermaßen zu erheblichen Nebenwirkungen, wie gastrointestinalen Störungen, Kopfschmerz, Schwindel, zentralnervöse Störungen.

Durchblutungsstörungen, als allgemeine Krankheitszeichen werden nach heutiger Auffassung einerseits durch arteriosklerotische Verengungen der Blutgefäße, andererseits durch pathologische Veränderungen der Blutfließeigenschaften verursacht, woraus durch die eingeschränkte Mikrozirkulation eine Mangelversorgung des Gewebes resultiert.
Dadurch kann es prinzipiell zu einer Einschränkung der Funktionsfähigkeit aller Gewebe- und Organsysteme kommen. Besonders betroffen sind die Funktionen von Herz, Gehirn, Augen, Innenohr und Muskelgewebe, insbesondere der Extremitäten. Erkrankungen der Herzkranzgefäße, die beispielsweise zum Herzinfarkt und plötzlichem Herztod führen können, wie Angina pectoris, werden einerseits verursacht durch Arteriosklerose und Spasmen, andererseits durch Hypertonie verbunden mit einer Verschlechterung der Blutfließeigenschaften. Hieraus resultiert generell ein Mangel an Substrat und Sauerstoff. Prinzipiell gilt das gleiche pathogenetische Prinzip für Durchblutungsstörungen des Auges, des Innenohrs und des Muskelgewebes, wobei bei verschlechterter Fluidität des Blutes die Mikrozirkulation gestört ist. [K.U. Benner, GIT Labor Medizin, 5, 295 - 302, (1985)].

Nach moderner Auffassung ergeben sich die nachfolgend genannten Krankheitsbilder aus der Wechselbeziehung zwischen dem morphologisch veränderten Blutgefäß und dem pathologisch veränderten Gefäßinhalt, wobei flüssige (Plasma) und korpuskuläre Bestandteile (Erythrozyten und Thrombozyten) zu verstehen sind.

Krankheiten des Gehirns sind
transistorisch-ischämische Attacke (TIA), PRIND (prolongierte-reversible-ischämisch-neurologische Defizite), HOP (hirnorganisches Psychosyndrom) mit Leistungsdefiziten bis hin zum Hirninfarkt (Schlaganfall).

Krankheiten der Gefäße sind
koronare Herzkrankheit, Angina pectoris sowie Hypertonie. Letztere ist per se mit verschlechterten Fließeigenschaften des Blutes verbunden und gilt zusätzlich als Risikofaktor für Schlaganfall und Herzinfarkt.

Krankheiten der Muskulatur sind
Claudicatio Intermittens, Ulcus cruris, Gangrän bis hin zur Nekrose mit Amputationsfolge, Morbus Raynaud. Am Auge sind Sehstörungen bis hin zur Erblindung, Am Ohr Ohrensausen, Hörstörungen, Hörsturz bis zum Hörverlust die Folge.

Es war bislang nicht bekannt, daß aufgrund der Wirkspezifität der Einzelsubstanzen, insbesondere der erfindungsgemäß verwendeten Dihydropyridine bzw. des Pentoxifyllins, eine gezielte Kombinationstherapie zur Behandlung, der als ursächlich anzusehenden verschlechterten Blutfließeigenschaften, durchgeführt werden kann, da ihre Verwendung bisher grundsätzlich alleine als Vasodilatator (Dihydropyridine) bzw. als durchblutungsförderndes Mittel (Pentoxifyllin) erfolgt.
(Rote Liste 1985, lfd. Nr. 26063 bzw. 36048, 36049).

Es war deshalb überraschend festzustellen, daß bei gleichzeitiger Anwendung der erfindungsgemäß ausgewählten Dihydropyridine mit Pentoxifyllin, dargestellt an Nifedipin als beispielhaftes Dihydropyridin, eine über die Wirkung der Einzelsubstanzen hinausgehende günstige Beeinflussung wichtiger Parameter der Blutfließeigenschaften erfolgte, nämlich in Form einer Verringerung der Erythrozytenaggregation, Erhöhung der Erythrozytenflexibilität und Hemmung der Thrombozytenaggregation, die über die Wirkung der Monosubstanz Pentoxifyllin hinausgeht, wobei die festgestellte überadditive Wirkung dem Dihydropyridin zugeschrieben werden muß, welches alleine auf diese Parameter keine bzw. kaum Wirkung ausübt.

Die erfindungsgemäßen Kombinationen von jeweils einem der beanspruchten Dihydropyridine mit Pentoxifyllin üben demnach dabei einen überadditiven Effekt aus, der sich bei der Kombinationstherapie in einer Verbesserung der Durchblutung der minderversorgten Gebiete niederschlägt.

Gleichzeitig wird die für Dihydropyridine an sich bekannte Vasoprotektion und der Herzschutz verstärkt. Damit kann in fortschrittlicher Weise eine Therapie durchgeführt werden zur Verbesserung der Blutfließeigenschaften, die fur die eingangs geschilderten Krankheiten mitverantwortlich sind oder als ursächlich zugesehen werden. Besonders vorteilhaft ist wegen der überadditiven Wirkung die Reduzierung der Wirkstoffkomponenten auf Dosen, die deutlich unter denen liegen, die zur jeweiligen Monotherapie erforderlich sind, was sich zwangsläufig auf die Verminderung der eingangs beschriebenen Nebenwirkungen positiv auswirkt. Die Dosen der Einzelkomponenten, die den erfindungsgemäßen Kombinationen zugrunde liegen, sind dabei je nach Art und Schweregrad der Erkrankung in Einzel- bzw. Tagesdosen anzupassen. Vornehmlich sind die Verhältnisse von Dihydropyridinen zu Pentoxifyllin 1:150 bis 1:2, wobei sich individuell für die ausgewählten Dihydropyridine, je nach Wirksamkeit, innerhalb dieses Bereiches besonders bevorzugte Verhältnisse ergeben. So sind für Nifedipin und Pentoxifyllin

Wirkstoffverhältnisse von 1:30 bis 1:10 sinnvoll. Für die Kombination Nimodipin mit Pentoxifyllin gilt 1:10 bis 1:4, für Nitrendipin mit Pentoxifyllin gilt 1:100 bis 1:10; für Nicardipin mit Pentoxifyllin 1:30 bis 1:2,5; für Felodipin mit Pentoxifyllin 1:40 bis 1:5. Sofern die genannten Dihydropyridine Salze bilden, können diese in den gleichen Verhältnissen verwendet werden.

Kennzeichnend für die Erfindung ist neben den Wirkstoffverhältnissen, daß die miteinander kombinierten Wirkstoffe gleichzeitig verabreicht werden, vorzugsweise in einer fixen Arzneiform. Dabei können, soweit erforderlich, neben den Wirkstoffen weitere Hilfsstoffe wie Füll- und Bindemittel, Träger, Schmier- und Gleitstoffe verwendet werden. Bevorzugte Arzneiformen sind dabei feste, oral zu verabreichende Formen wie Tabletten, Filmtabletten, Kapseln, jedoch auch rektal zu verabreichende Formen, wie beispielsweise Suppositorien. In bedrohlichen Fällen sind parenteral zu verabreichende Formen, insbesondere für den Bereich der Klinik, vorzuziehen, welche als Trägermedium mit den Wirkstoffen kompatible Verdünnungsmittel enthalten, wie zum Beispiel organische Lösungsmittel. Selbstverständlich kommen auch Lösungen als fixe Kombinationen zur oralen Verabreichung infrage, z. B. in Form von Tropfen.

Erfindungsgemäß können diese Kombinationen auch in nebeneinanderliegenden, getrennten Arzneiformen vorliegen, insbesondere dann, wenn die Arzneiformen von den räumlichen Abmessungen her eine Applikation erschweren. Dies gilt besonders für die oralen Formen, da häufig bei älteren Patienten eine Abneigung gegen große Tabletten und Kapseln vorherrscht. Zwingend ist, daß die getrennt, nebeneinander vorliegenden Arzneiformen zur zeitlich gemeinsamen Einnahme hergerichtet sind. Dabei können auch unterschiedliche Formen, z. B. Tablette und Kapsel, nebeneinander vorliegen. Für die länger andauernde Therapie ist die regelmäßige Einnahme erfolgsbestimmend. Dazu eignen sich in der Regel nur orale und rektale Arzneiformen, wobei diese, soweit sie in Siegelblistern vorliegen, auf einer Seite eine Zahlen- oder Tagesangabe tragen, die dem Patienten die Einnahmekontrolle erlauben und damit die Compliance sichern.

Die Verabreichung der erfindungsgemäßen Kombination beider Stoffe zur gleichzeitigen Einnahme im Sinne dieser Erfindung, garantiert somit eine hohe Therapie- und Patientencompliance.

Die günstige Wirkung der erfindungsgemäßen Kombinationen, die einen überadditiven Effekt ausüben, läßt sich in der nachfolgend beschriebenen Untersuchung, am Beispiel der Kombination Nifedipin mit Pentoxifyllin, nachweisen.

<u>Ausführungsbeispiel:</u>

Es wurde Blut von zehn Patienten (Zustand nach Hirninfarkt, peripherer arterielle Verschlußkrankheit, ischämische Herzerkrankung) abgenommen, das nach der Methode DODDS-DORMANDY, Brit. Med. Journ., Nov. 1979, S. 1186 - 1187, eine pathologische Filtrabilität aufwies. Ausschlußkriterium war eine Erythrozytenfiltrabilität besser als 0,53. Es war sichergestellt, daß die Patienten vor Blutentnahme drei Tage keine Rheologika bzw. Thrombozytenaggregationshemmer eingenommen hatten.

<u>Untersuchungsparameter:</u> Erythrozytenflexibilität, Erythrozytenaggregation, Thrombozytenaggregation (ADP-induziert).

<u>Meßmethoden:</u>

1. <u>Erythrozytenflexibilität:</u>

Methode zur Filtrabilität nach DODDS-DORMANDY unter Verwendung von 5 µm Nucleopor-Filter.

2. <u>Erythrozytenaggregation:</u>

Myrenne-Aggreometer (Typ MAI, Fa. Myrenne, 5106 Roetgen) Methode nach Kiesewetter et al., Biomed. Technik, <u>27</u>, S. 209, 1982.

3. <u>Thrombozytenaggregation:</u>

Methode nach BORN, J. Physiol. <u>168</u>, S. 178 - 195, (1963), unter Verwendung eines Bio/Data-Trombozyten Aggregations-Profiler (Typ PAP4).

<u>Prüfpräparate:</u>

1. Physiologische Natriumchloridlösung (Placebo)
2. Pentoxifyllin-Lösung, 10 µg/ml
3. Pentoxifyllin-Lösung, 1 µg/ml
4. Pentoxifyllin-Lösung, 0,1 µg/ml
5. Mischung einer physiologischen Natriumchloridlösung mit Ethylalkohol (Placebo).
6. Nifedipin-Lösung 1,5 µg/ml

7. Nifedipin-Lösung 0,15 µg/ml
8. Nifedipin-Lösung 0,015 µg/ml
9. Kombination aus Lösung 2 und 6 ( 10 µg/ml - 1,5 µg/ml)
10. Kombination aus Lösung 3 und 7 ( 1 µg/ml - 0,15 µg/ml)
11. Kombination aus Lösung 4 und 8 ( 0,1 µg/ml - 0,015 µg/ml)

Versuchsablauf:

Zum direkten Vergleich wurden Blutproben mit einer Mischung aus Lösung 1 und 5 (Placebo) 30 Minuten bei 37°C inkubiert und gemessen. Entsprechend wurden Blutproben unter Zugabe von Wirkstoff-Lösungen der vorgenannten Tabelle, d. h. der Einzelwirkstoffe bzw. deren Kombinationen, jeweils in den obengenannten Konzentrationen behandelt.

Probenaufbereitung:

Um plättchenreiches Plasma zur Bestimmung der Thrombozytenaggregationshemmung zu erhalten, wurden 4mal 2,5 ml Blut nach Behandlung zentrifugiert.

Um plättchenfreies Plasma, zur Bestimmung der Erythrozytenflexibilität zu erhalten, wurden 10 ml Blut nach Behandlung bei 3000 G zentrifugiert.

Zur Bestimmung der Erythrozytenaggregationshemmung wurde Blut in üblicher Weise antikoaguliert. Die Aggregation wurde bei einem Hämatokrit von 45 % photometrisch als Schergrad bestimmt, bei dem eine vollständige Dispersion der Erythrozytenaggregate auftritt (Methode nach Ernst, Labor Praxis in der Medizin, April 1984, S. 18 - 21).

Die Bestimmung der Einzelparameter erfolgte jeweils dreifach.

Auswertemethode:

Die gemessenen Daten wurden statistisch nach WILCOXON + WILCOX auf Signifikanz geprüft.

Ergebnisse:

In den Abbildungen 1A bis 3C sind die Ergebnisse in Form von Balkendiagrammen wiedergegeben.

Wertung der Ergebnisse:

1. Erythrozytenflexibilität

Aus Abb. 1A geht hervor, daß Pentoxifyllin allein erst in einer Konzentration von 10 µg/ml Probe gegenüber der Placeboprobe eine schwach signifikante ($p < 0,05$) Erhöhung der Erythrozytenflexibilität ergab.

Nifedipin allein, Balkendiagramm Abb. 1B führte erst in der höchsten Konzentration von 1,5 µg/ml zu dem oben bereits beschriebenen Effekt.

Aus Abb. 1C ist ersichtlich, daß die Kombination aus Nifedipin und Pentoxifyllin, bereits in einer Konzentration, die um eine 10er Potenz niedriger liegt, als die höchsten Konzentrationen der Einzelkomponenten, eine hochsignifikante ($p < 0,01$) Erhöhung der Erythrozytenflexibilität ergibt.

2. Erythrozytenaggregation

Abb. 2A zeigt, daß Pentoxifyllin allein eine Aggregationshemmung bei einer Konzentration von 10 µg/ml Probe ($p < 0,05$) induziert.

Nifedipin allein, Abb. 2B, zeigte keinerlei aggregationshemmenden Effekt bei den geprüften Konzentrationen. Es wurde im Gegenteil eine Verstärkung der Aggregationsneigung gemessen.

Aus Abb. 2C ist ersichtlich, daß bereits die mittlere Konzentration der Kombination eine signifikante und die höchste Konzentration eine hochsignifikante ($p < 0,01$) Verringerung der Erythrozytenaggregation hervorruft.

3. Thrombozytenaggregation (ADP-induziert)

Abb. 3A zeigt, daß lediglich bei der höchsten Pentoxifyllin-Konzentration von 10 µg/ml eine hochsignifikante ($p < 0,01$) Abnahme der Thrombozytenaggregation gegenüber Placebo bzw. den anderen Konzentrationen erfolgt.

Bei alleiniger Gabe von Nifedipin , Abb. 3B, wurde bei allen Konzentrationen kein signifikanter Effekt gemessen. Erst die Kombination aus Pentoxifyllin und Nifedipin, Abb. 3C, zeigt bereits bei mittleren Konzentrationen gegenüber Placebo eine signifikante Abnahme der Thrombozytenaggregation.

Die Ergebnisse mit den Wirkstoffkombinationen zeigen signifikante Verbesserungen der geprüften Parameter bereits in Dosierungen, bei denen die Einzelwirkstoffe nicht bzw. nur schwach wirksam sind. Selbst in den untersten Konzentrationen der Wirkstoffkombination, die Konzentrationen darstellen, wie sie in vivo als subtherapeutische Plasmaspiegel auftreten, werden relevante Effekte gesehen.

Die Ergebnisse veranschaulichen in eindrucksvoller Weise, daß durch die erfindungsgemäße Kombination, am Beispiel des Dihydropyridins Nifedipin mit Pentoxifyllin, gegenüber den Einzelsubstanzen eine therapeutisch relevante Verbesserung der gemessenen Parameter eintritt. Dies hat unmittelbaren Einfluß auf die Verbesserung der Blutfließeigenschaften, die, wie eingangs beschrieben, ursächlich in die Pathogenese der verschiedenen schweren Krankheitsbilder eingreifen. Darüber hinaus ist von besonderer Bedeutung, daß die Konzentrationen der verwendbaren Wirkstoffkombinationen, deutlich gegenüber den denkbaren Einzeldosen, mit den beschriebenen Nebenwirkungen, gesenkt werden können, und damit eine therapiegerechte Dauerapplikation bei verringerter Belastung des Organismus ermöglichen.

**Patentansprüche**

1. Pharmazeutisches Kombinationspräparat zur Behandlung von Durchblutungsstörungen, dadurch **gekennzeichnet,** daß es als Wirkstoff A ein Dihydropyridin mit calciumantagonistischer Wirkung der allgemeinen Formel

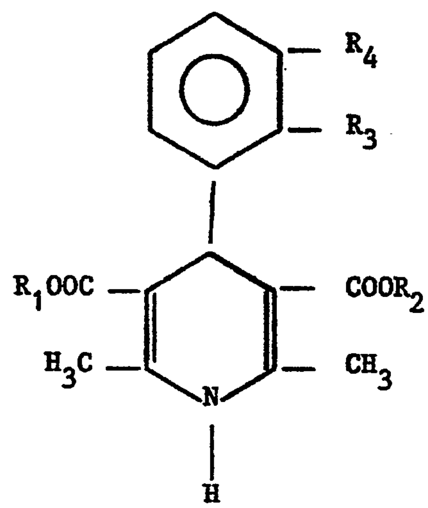

in der
$R_1$ eine $CH_3$-O-$CH_2$-$CH_2$- oder $CH_3$-Gruppe, $R_2$ eine

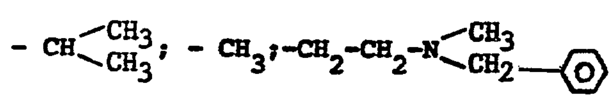

oder $CH_3$–$CH_2$-Gruppe;
$R_3$ H, $NO_2$ oder Cl und
$R_4$ $NO_2$, H oder Cl
bedeuten, und Pentoxifyllin als Wirkstoff B neben üblichen Träger- und Zusatzstoffen enthält, wobei die Mengenverhältnisse der Wirkstoffe A und B, 1:150 bis 1:2 betragen.

2. Pharmazeutisches Kombinationspräparat nach Anspruch 1, dadurch gekennzeichnet, daß es als Wirkstoff A Nifedipin enthält.

3. Pharmazeutisches Kombinationspräparat nach Anspruch 1, dadurch gekennzeichnet, daß es als Wirkstoff A Nimodipin enthält.

4. Pharmazeutisches Kombinationspräparat nach Anspruch 1, dadurch gekennzeichnet, daß es als Wirkstoff A Nitrendipin enthält.

5. Pharmazeutisches Kombinationspräparat nach Anspruch 1, dadurch gekennzeichnet, daß es als Wirkstoff A Nicardipin enthält.

6. Pharmazeutisches Kombinationspräparat nach Anspruch 1, dadurch gekennzeichnet, daß es als Wirkstoff A Felodipin enthält.

7. Pharmazeutisches Kombinationspräparat nach Anspruch 2 zur Behandlung von Durchblutungsstörungen, dadurch gekennzeichnet, daß es die Wirkstoffe Nifedipin und Pentoxifyllin in einem Verhältnis von 1:30 bis 1:10 enthält.

8. Pharmazeutisches Kombinationspräparat nach Anspruch 3 zur Behandlung von Durchblutungsstörungen, dadurch gekennzeichnet, daß es die Wirkstoffe Nimodipin und Pentoxifyllin in einem Verhältnis von 1:10 bis 1:4 enthält.

9. Pharmazeutisches Kombinationspräparat nach Anspruch 4 zur Behandlung von Durchblutungsstörungen, dadurch gekennzeichnet, daß es die Wirkstoffe Nitrendipin und Pentoxifyllin in einem Verhältnis von 1:100 bis 1:10 enthält.

10. Pharmazeutisches Kombinationspräparat nach Anspruch 5 zur Behandlung von Durchblutungsstörungen, dadurch gekennzeichnet, daß es die Wirkstoffe Nicardipin und Pentoxifyllin in einem Verhältnis von 1:30 bis 1:2,5 enthält.

11. Pharmazeutisches Kombinationspräparat nach Anspruch 6 zur Behandlung von Durchblutungsstörungen, dadurch gekennzeichnet, daß es die Wirkstoffe Felodipin und Pentoxifyllin in einem Verhältnis von 1:40 bis 1:10 enthält.

12. Pharmazeutisches Kombinationspräparat nach Anspruch 1 bis 11, dadurch gekennzeichnet, daß es die Wirkstoffe gemeinsam als fixe Kombination in Darreichungsformen zur rektalen oder oralen oder parenteralen Applikation enthält, erforderlichenfalls unter Verwendung von einem oder mehreren Hilfsstoffen.

13. Kombinationspräparat nach Anspruch 1 bis 11, dadurch gekennzeichnet, daß beide Wirkstoffe in gleichartigen, getrennten Arzneiformen zur zeitlich gemeinsamen Verabreichung vorliegen.

14. Kombinationspräparat nach Anspruch 1 bis 11, dadurch gekennzeichnet, daß beide Wirkstoffe in getrennten, unterschiedlichen Arzneiformen zur zeitlich gemeinsamen Verabreichung vorliegen.

**Claims**

1. Pharmaceutical combination preparation for the treatment of circulatory disturbances, characterized in that it contains, as active compound A, a dihydropyridine having calcium antagonist action, of the general formula

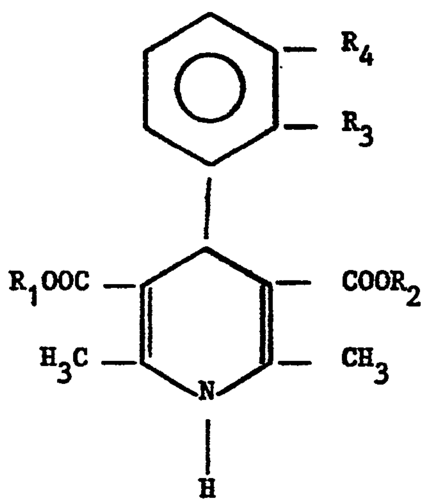

in which
$R_1$ denotes a $CH_3-O-CH_2-CH_2-$ or $CH_3-$ group,
$R_2$ denotes a

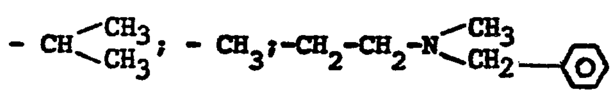

or $CH_3-CH_2-$group;
$R_3$ denotes H, $NO_2$ or Cl and
$R_4$ denotes $NO_2$, H or Cl
and pentoxifylline as active compound B in addition to customary excipients and additives, the proportions of the active compounds A and B being 1:150 to 1:2.

2. Pharmaceutical combination preparation according to Claim 1, characterized in that it contains nifedipine as active compound A.

3. Pharmaceutical combination preparation according to Claim 1, characterized in that it contains nimodipine as active compound A.

4. Pharmaceutical combination preparation according to Claim 1, characterized in that it contains nitrendipine as active compound A.

5. Pharmaceutical combination preparation according to Claim 1, characterized in that it contains nicardipine as active compound A.

6. Pharmaceutical combination preparation according to Claim 1, characterized in that it contains felodipine as active compound A.

7. Pharmaceutical combination preparation according to Claim 2 for the treatment of circulatory disturbances, characterized in that it contains the active compounds nifedipine and pentoxifylline in a ratio of 1:30 to 1:10.

8. Pharmaceutical combination preparation according to Claim 3 for the treatment of circulatory disturbances, characterized in that it contains the active compounds nimodipine and pentoxifylline in a ratio of 1:10 to 1:4.

9. Pharmaceutical combination preparation according to Claim 4 for the treatment of circulatory disturbances, characterized in that it contains the active compounds nitrendipine and pentoxifylline in a ratio of 1:100 to 1:10.

10. Pharmaceutical combination preparation according to Claim 5 for the treatment of circulatory disturbances, characterized in that it contains the active compounds nicardipine and pentoxifylline in a ratio of 1:30 to 1:2.5.

11. Pharmaceutical combination preparation according to Claim 6 for the treatment of circulatory disturbances, characterized in that it contains the active compounds felodipine and pentoxifylline in a ratio of 1:40 to 1:10.

12. Pharmaceutical combination preparation according to Claim 1 to 11, characterized in that it contains the active compounds together as a fixed combination in administration forms for rectal or oral or parenteral administration, if necessary using one or more auxiliaries.

13. Combination preparation according to Claim 1 to 11, characterized in that both active compounds are present in similar, separated pharmaceutical forms for periodic joint administration.

14. Combination preparation according to Claim 1 to 11, characterized in that both active compounds are present in separate, different pharmaceutical forms for periodic joint administration.

## Revendications

1. Préparation pharmaceutique combinée pour traiter les troubles de la circulation, caractérisée en ce que l'on utilise comme substance active A une dihydropyridine avec effet antagoniste du calcium suivant la formule générale

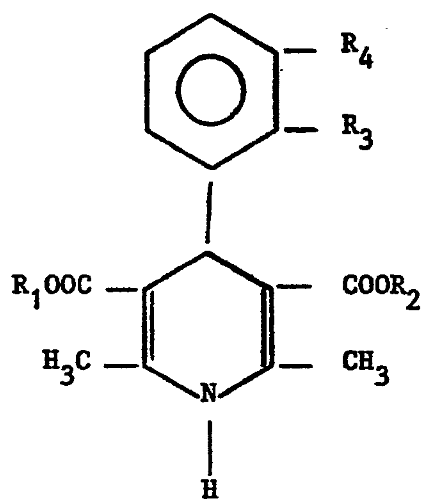

dans laquelle

$R_1$ représente un groupe $CH_3-O-CH_2-CH_2-$ ou $CH_3-$

$R_2$ représente un groupe

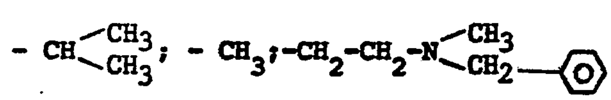

ou un groupe $CH_3-CH_2-$

$R_3$ représente H, $NO_2$ ou Cl et
$R_4$ représente $NO_2$, H ou Cl
et de la pentoxyfylline comme substance B ainsi que des supports et des additifs usuels avec un rapport des quantités de substances A et B allant de 1:150 à 1:2.

2. Préparation pharmaceutique combinée selon la revendication 1, caractérisée en ce qu'elle contient la nifédipine comme substance active A.

3. Préparation pharmaceutique combinée selon la revendication 1, caractérisée en ce qu'elle contient la nimodipine comme substance active A.

4. Préparation pharmaceutique combinée selon la revendication 1, caractérisée en ce qu'elle contient la nitrendipine comme substance active A.

5. Préparation pharmaceutique combinée selon la revendication 1, caractérisée en ce qu'elle contient la nicardipine comme substance active A.

6. Préparation pharmaceutique combinée selon la revendication 1, caractérisée en ce qu'elle contient la félodipine comme substance active A.

7. Préparation pharmaceutique combinée selon la revendication 2 pour le traitement des troubles de la circulation, caractérisée en ce qu'elle contient les substances actives nifédipine et pentoxyfillyne avec un rapport de 1:30 à 1:10.

8. Préparation pharmaceutique combinée selon la revendication 3 pour le traitement des troubles de la circulation, caractérisée en ce qu'elle contient les substances actives nimodipine et pentoxyfillyne avec un rapport de 1:10 à 1:4.

9. Préparation pharmaceutique combinée selon la revendication 4 pour le traitement des troubles de la circulation, caractérisée en ce qu'elle contient les substances actives nitrendipine et pentoxyfillyne avec un rapport de 1:100 à 1:10.

10. Préparation pharmaceutique combinée selon la revendication 5 pour le traitement des troubles de la circulation, caractérisée en ce qu'elle contient les substances actives nicardipine et pentoxyfillyne avec un rapport de 1:30 à 1:2,5.

11. Préparation pharmaceutique combinée selon la revendication 6 pour le traitement des troubles de la circulation, caractérisée en ce qu'elle contient les substances actives félodipine et pentoxyfillyne avec un rapport de 1:40 à 1:10.

12. Préparation pharmaceutique combinée selon les revendications 1 à 11, caractérisée en ce qu'elle contient les substances actives groupés sous forme de combinaisons fixes qui permettent l'administration rectale, orale ou parentérale, éventuellement avec utilisation d'un ou plusieurs adjuvant(s).

13. Préparation pharmaceutique combinée selon les revendications 1 à 11, caractérisée en ce que les deux substances se présentent sous forme de médicaments séparés de même nature, en vue de leur administration commune simultanée.

14. Préparation pharmaceutique combinée selon les revendications 1 à 15, caractérisée en ce que les deux substances se présentent sous forme de médicaments séparés différents, en vue de leur administration commune simultanée.

Abb. 1A

Erythrozytenflexibilität

Pentoxifyllin

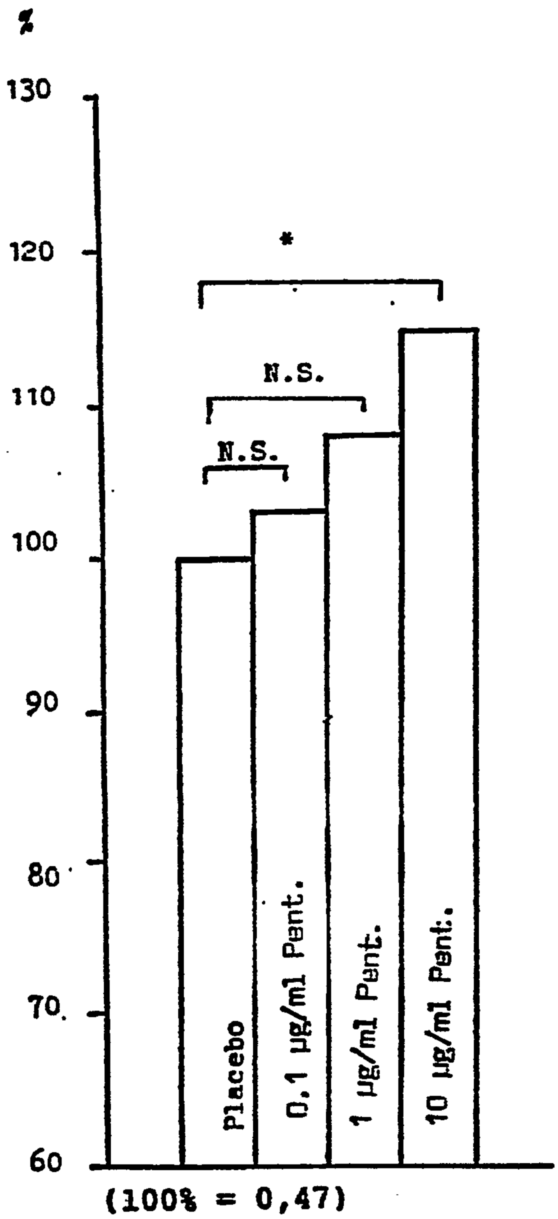


* p < 0,05

N.S. = nicht signifikant

Pent. = Pentoxifyllin

Abb. 1B

Erythrozytenflexibilität

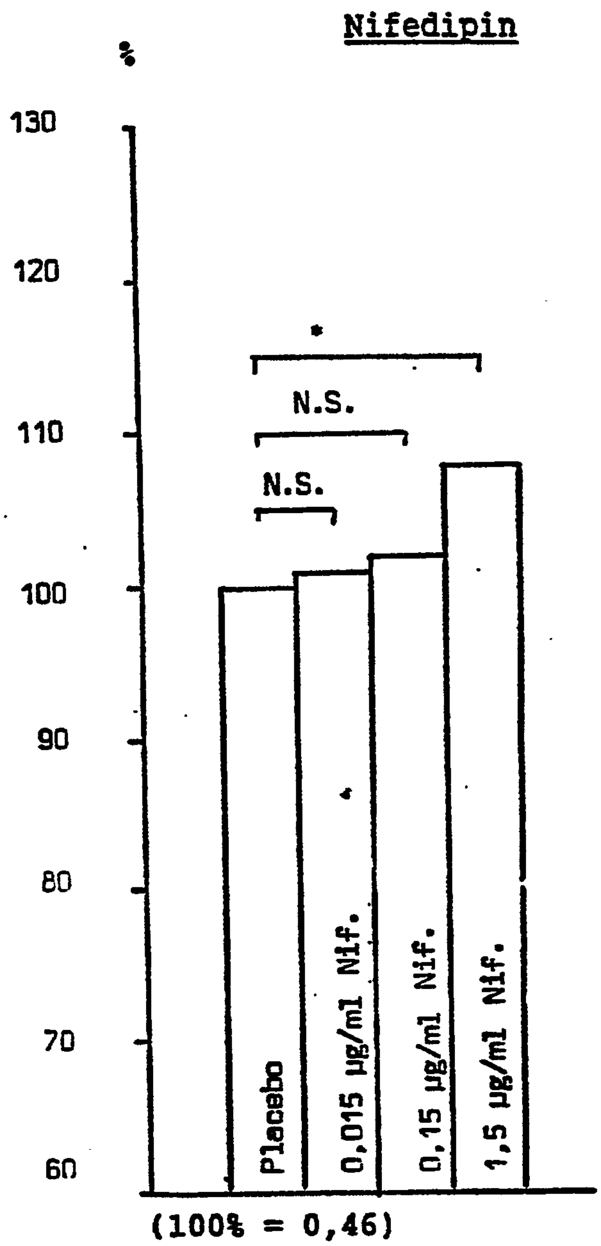


* p < 0,05

N.S. = nicht signifikant

Nif. = Nifedipin

Abb. 1C

Erythrozytenflexibilität

Pentoxifyllin + Nifedipin

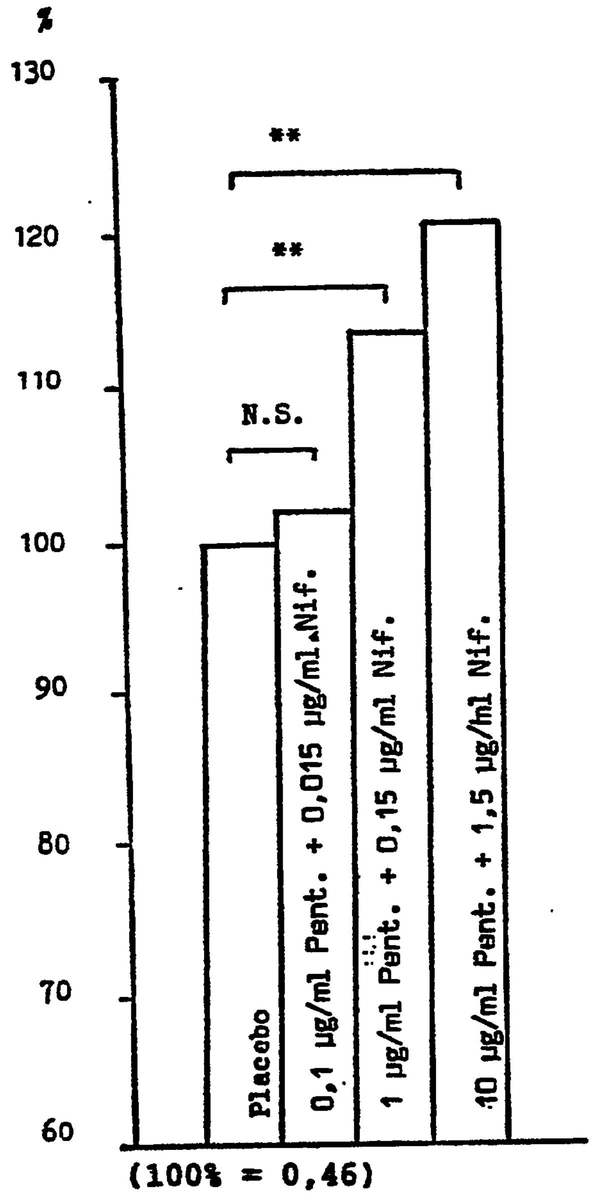


** $p < 0{,}01$
N.S. = nicht signifikant

Pent. = Pentoxifyllin
Nif. = Nifedipin

Abb. 2A

Erythrozytenaggregation

Pentoxifyllin

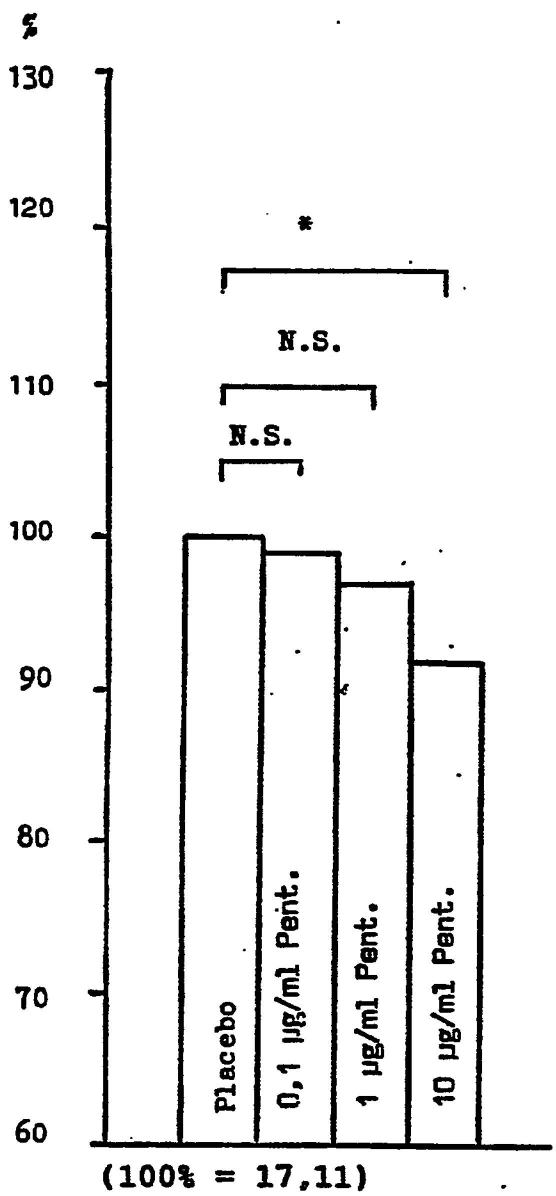


* p < 0,05

N.S. = nicht signifikant

Pent. = Pentoxifyllin

Abb. 2B

Erythrozytenaggregation

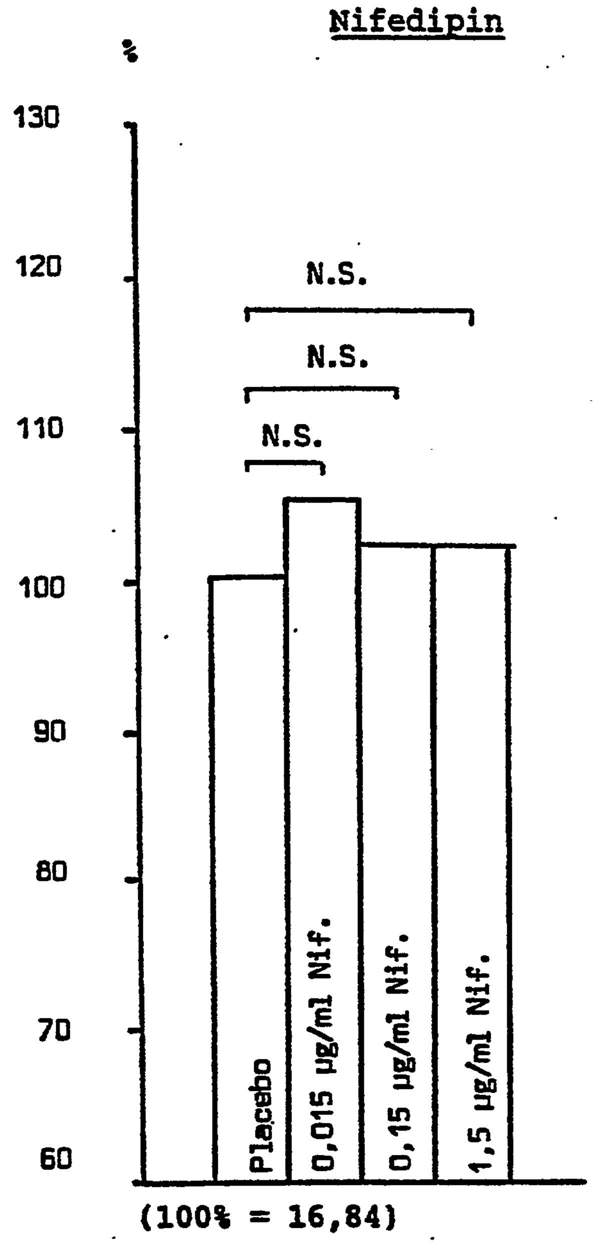


N.S. = nicht signifikant

Nif. = Nifedipin

Abb. 2C

Erythrozytenaggregation

Pentoxifyllin + Nifedipin

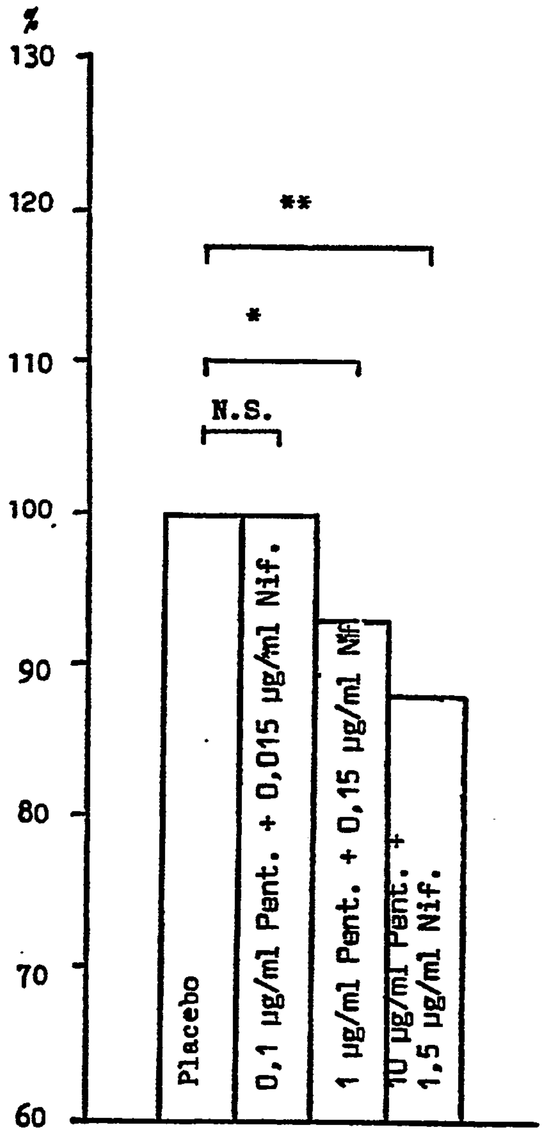


(100% = 17,36)

* p < 0,05
** p < 0,01
N.S. = Nicht signifikant

Pent. = Pentoxifyllin
Nif. = Nifedipin

Abb. 3A

Thrombozytenaggregation
(ADP-induziert)

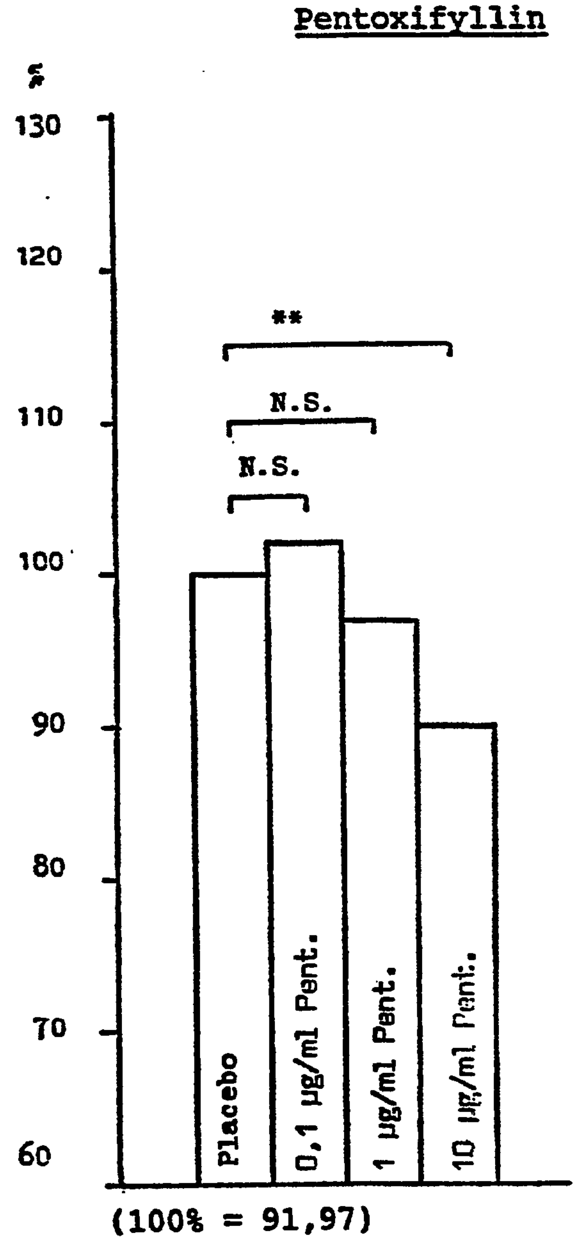


Pent. = Pentoxifyllin

** p < 0,01
N.S. = Nicht signifikant

Abb. 3B

Thrombozytenaggregation
(ADP-induziert)

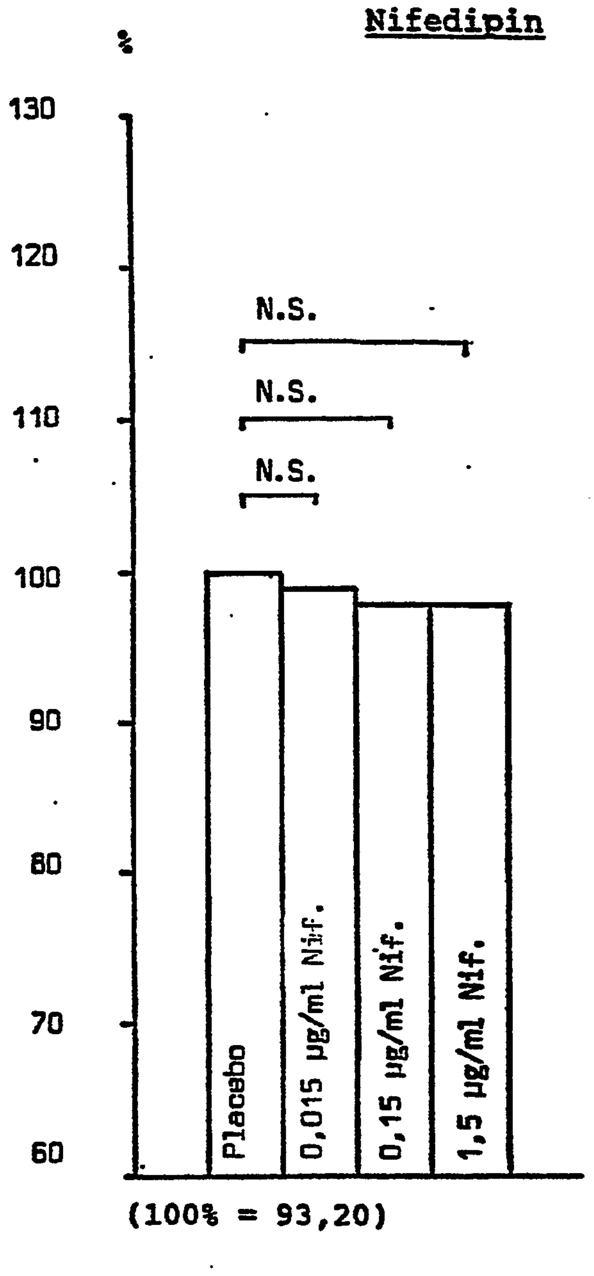


N.S. = Nicht signifikant

Nif. = Nifedipin

Abb. 3C

Thrombozytenaggregation
(ADP-induzierte)

Pentoxifyllin + Nifedipin

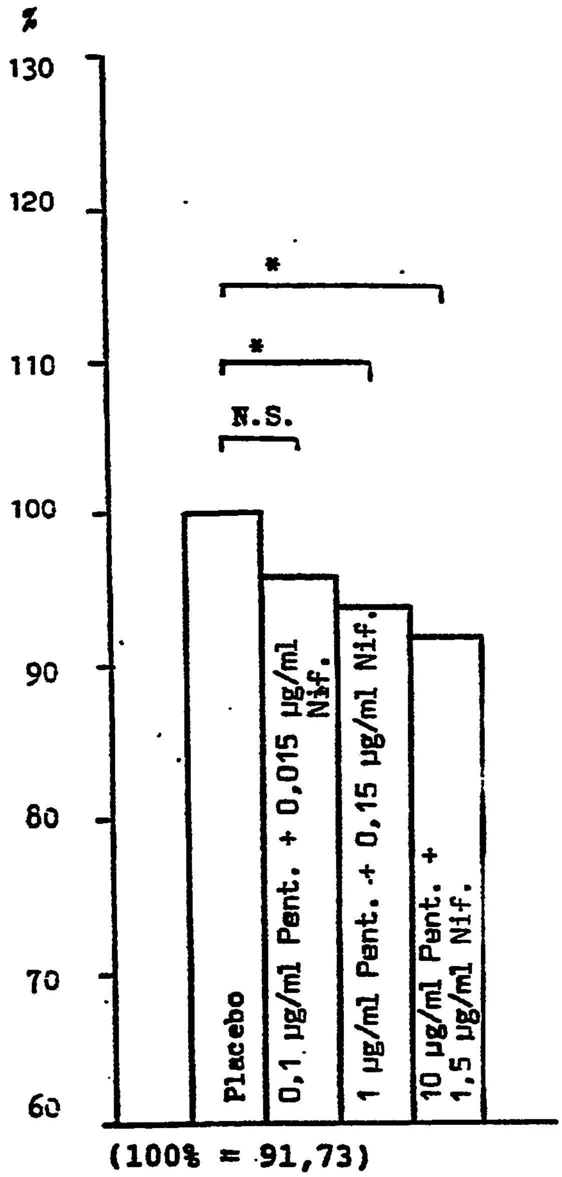


* $p < 0,05$

N.S. = Nicht signifikant

Pent. = Pentoxifyllin
Nif. = Nifedipin